# EUROPEAN PATENT SPECIFICATION

(11) **EP 1 846 145 B1**
(45) Date of publication and mention of the grant of the patent: **14.04.2010**
(21) Application number: 06713723.2
(22) Date of filing: 08.02.2006
(51) Int. Cl.: B01F 5/24, A61K 9/14

(54) **MIXING APPARATUS AND METHOD**
MISCHVORRICHTUNG UND VERFAHREN
MÉLANGEUR ET PROCÉDÉ

(30) Priority: 09.02.2005 JP 2005032835
(43) Date of publication of application: 24.10.2007
(73) Proprietor: Sumitomo Chemical Company, Limited, Tokyo 104-8260 (JP)
(72) Inventor: UEDA, Nobuhito, 263 0042 (JP)
(74) Representative: Vossius & Partner
(86) International application number: PCT/JP2006/302582
(87) International publication number: WO 2006/085666

(56) References cited:
- FR-A- 793 714
- GB-A- 581 849
- US-A- 1 574 210
- US-A- 4 746 222

## Description

The present invention relates to a mixing apparatus for mixing plural kinds of particulate materials.

As a mixing apparatus for mixing plural kinds of particulate materials by using a falling force (or gravitational force), a mixing apparatus described in Japanese Utility Model Kokai Publication No. S53-90274 is known. According to this mixing apparatus, plural kinds of particulate materials are charged from the top of the apparatus and mixed while falling in the apparatus, and thus mixed particulate materials are discharged from the bottom of the apparatus.

GB 581,849 A, which discloses a mixing apparatus in accordance with the preamble of claim 1 and a method in accordance with the preamble of claim 5, relates to the mixing of solid materials by causing them to commingle in flow making use of the action of gravity. FR 793,714 A discloses a mixing apparatus for venting and conditioning grain. US 4,746,222 A discloses an apparatus for the mixing and cooling of hot, particulate matter, comprising a plurality of enclosed, vertically spaced apart, converging vessels through which the material to be mixed and cooled is flowed.

With respect to such mixing apparatus for mixing plural kinds of particulate materials by using a falling force, it is preferable in view of an operating efficiency to shorten a period from the beginning to the end of discharge of the mixed particulate materials. We studied in the mixing apparatus for mixing plural kinds of particulate materials by using a falling force, a means which can shorten the period from the beginning to the end of discharge of the mixed particulate materials. As a result, in the mixing apparatus for mixing plural kinds of the particulate materials by using the falling force, we found that by humidifying a space for mixing the particulate materials, the period from the beginning to the end of discharge of the mixed particulate materials is shortened.

Therefore, the present invention is as follows:
[1] A mixing apparatus for mixing plural kinds of particulate materials by using a falling force (or gravitational force), wherein the mixing apparatus comprises means for humidifying a space for mixing the particulate materials, characterized in that the humidifying means is adapted so that the relative humidity in the space for mixing is made at 50% or more;
[2] The mixing apparatus according to [1], characterized in that the mixing apparatus comprises at least one set of a diffluent part for dividing a passage through which the particulate materials fall and a confluent part for merging passages through which the particulate materials fall;
[3] A method for mixing plural kinds of particulate materials by using a falling force, wherein the method is carried out in a humidified space for mixing the particulate materials, characterized in that the relative humidity in the humidity space is made at 50% or more.
[4] The method according to [3], characterized in that the plural kinds of the particulate materials comprise at least one kind of a particulate material containing a pharmaceutical component, an agrochemical component or a fertilizer component.

The mixing apparatus of the present invention is the mixing apparatuses for mixing plural kinds of particulate materials by using a falling force, and provided with means for humidifying a space for mixing the particulate materials.

At first, the structure of the mixing apparatus for mixing plural kinds of the particulate materials is described. The mixing apparatuses for mixing plural kinds of the particulate materials is generally in the form of a tubular article having a charging port for charging the particulate materials at the top of the apparatus, a discharging port of the particulate materials at the bottom of the apparatus, and a space between the charging port and the discharging port for mixing the particulate materials. The space for mixing the particulate materials is generally constructed with a distributing part for distributing a flow of the particles and a collecting part for getting flows of the particles together.

In the course of the movement of the plural kinds of the particulate materials charged from the charging port of the mixing apparatus downward by falling under the effect of gravity, the particulate materials are distributed and collected together, so that the mixing of the particulate materials is conducted.

Next, the structure for distributing and collecting the particulate materials in the space for mixing the particulate materials is described. This structure is provided with a plate(s) or the like to change the passage(s) of the particulate materials during falling of the particulate materials from the top to the bottom of the mixing apparatus of the present invention. Thus, when the plural kinds of the particulate materials are fallen down from the top of the mixing apparatus, the plural kinds of the articulate materials are mixed with each other by repeating the distribution and collection of the particulate materials totally or partially in the space for mixing the particulate materials. As an example of the structure of the space for mixing the particulate materials in the mixing apparatus, it is considered to provide the structure with plural sets of a diffluent part for dividing a passage through which the particulate materials fall and a confluent part for merging passages through which the particulate materials fall.
Fig. 1 is a cross-sectional view showing a schematic construction of a mixing apparatus in one embodiment of the present invention.
Fig. 2 is a cross-sectional perspective view showing a schematic structure of a diffluent part and a confluent part in the mixing apparatus of Fig. 1.
Fig. 3 is a top view showing a schematic structure of the diffluent part in the mixing apparatus of Fig. 1.
Fig. 4 is a perspective view showing a schematic structure of a storage tank which may be located on or above the mixing apparatus of Fig. 1.
Fig. 5 is a cross-sectional view showing a schematic construction of a mixing apparatus used in examples.

### Explanation of letters or numerals

1 ... diffluent part
2 ... confluent part
3 ... discharging part chute
4a, 4b, 4c, 4d ... outlet of funnel part
5 ... adjusting umbrella
6 ... adjusting umbrella supporting arm
7 ... dispersing umbrella
8 ... outlet of confluent part
9 ... guiding chute
10 ... humidifying means
10a ... emitting port of humidified air
11a, 11b ... storage tank funnel portion
12a, 12b ... outlet of storage tank funnel portion
13 ... damper

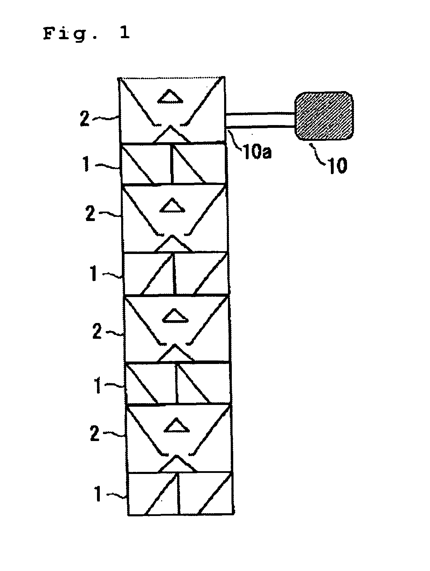

A more specific structure as to the space of the mixing apparatus for mixing the particulate materials according to the present invention is illustrated in Fig. 1. The illustrated structure is described below.

Fig. 1 shows a side elevational view of the structure for mixing the particulate materials. This structure is in the form of a rectangular tube having a square section, and constructed with plural stages which are composed of the diffluent part for dividing a passage through which the particulate materials fall and the confluent part for merging passages through which the particulate materials fall, alternately. Fig. 1 shows the structure constructed with four stages of the diffluent part 1 and the confluent part 2 each.

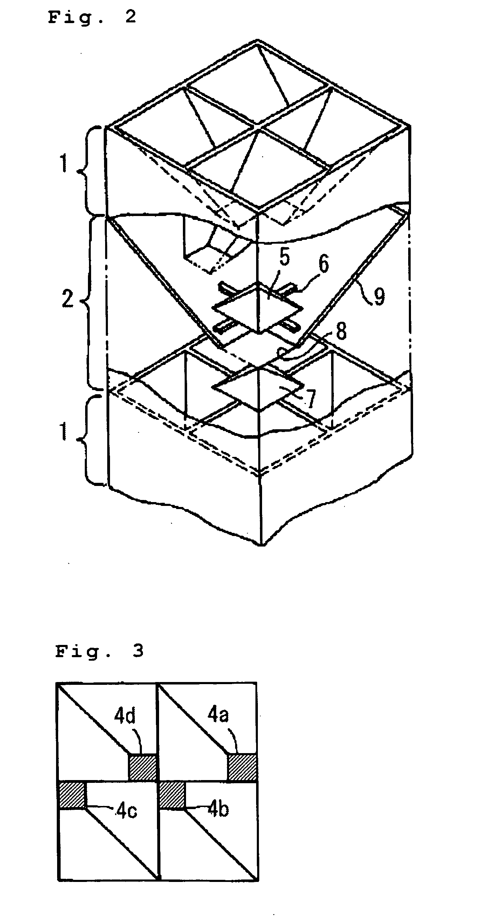

The diffluent part 1, as shown in Fig. 2, is divided at the top plane into four square sections as a charging port of a certain group of the particulate materials, and each of the sections has a funnel part of which lower portion is made narrower similarly to a funnel. Then, as shown in Fig. 3, outlets 4a, 4b, 4c and 4d for the respective funnel parts are located symmetrically about a point which is a center of the set of the four square sections. Specifically, the outlets 4b and 4d are located near the center in the two sections in the diagonal relationship, and the outlets 4a and 4c are located distal to the center in the remaining two sections.

The confluent part 2 has a guiding chute 9 at its upper portion of which shape becomes narrower toward an outlet 8, and an adjusting umbrella (or pyramidal member) 5 is located inside the guiding chute 9. The adjusting umbrella 5, though it is not necessary for the present invention, is supported with arms or supports 6 and located at a distance from the wall of the guiding chute 9 to prevent the guiding chute 9 from blockage and to disperse the group of the particulate materials discharged from the outlets 4b and 4d of the above diffluent part 1. The group of the particulate materials dispersed by the adjusting umbrella 5 as well as the group of the particulate materials discharged from the outlets 4a and 4c of the above diffluent part 1 are collected by the guiding chute 9 and fall from the outlet 8.

Further, other diffluent part of the next stage is located below the above confluent part 2. The particulate materials collected by the guiding chute 9 are divided into four funnel parts again by the diffluent part of the next stage. Additionally, a dispersing umbrella 7 may be located immediately below the outlet 8 to divide into the four funnel parts of the diffluent part, smoothly.

Therefore, the particulate materials are mixed in the structure for mixing the particulate materials by dividing its passage by the diffluent part 1 and merging the divided passages into one in the confluent part 2, and repeating the dispersion and the collection of the particulate materials.

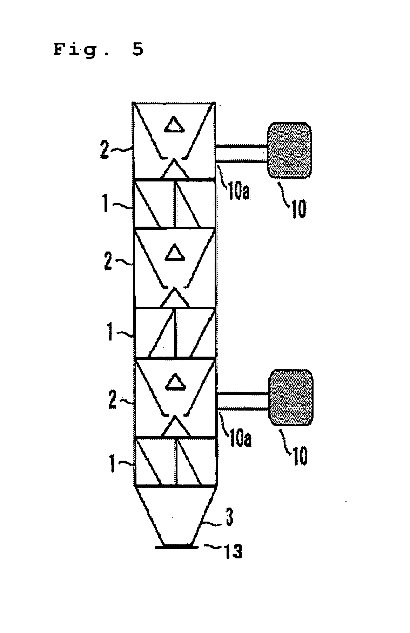

The mixing apparatus of the present invention is achieved by providing the mixing apparatus for mixing the plural kinds of the particulate materials with means for humidifying a space for mixing the particulate materials. A specific example of this humidifying means is a humidifier having a port for emitting humidified air to the space. There may be many types of the humidifier, such as a heating type (or steaming type) in which steam is generated by heating water, an ultrasonic type in which small water droplets (mist) are generated by an ultrasonic vibrator, and a mixed type thereof, and the present invention can utilize any type of the humidifier as the specific humidifying means. The ultrasonic type is preferable since it has a stable effect of humidification even if a temperature of air is low and it can prevent dew condensation inside the mixing apparatus. It is sufficient for the humidifying means to humidify the space for mixing the particulate materials in the mixing apparatus of the present invention, so that the emitting port of the humidified air has only to be connected to the space for mixing the particulate materials, and it is not necessary to put the humidifier next to the space for mixing the particulate materials (see, for example, the emitting port 10a in Fig. 1). Two or more humidifier may be used, if needed (see, for example, Fig. 5). In view of efficiency of humidification, the emitting port of the humidified air is preferably positioned above that space, in other words, in the vicinity of the charging port for the particulate materials of the mixing apparatus for mixing the plural kinds of particulate materials by using a falling force.

The mixing apparatus of the present invention is generally used as follows.

At first, the space for mixing the particulate materials in the mixing apparatus of the present invention is humidified by the humidifying means. Next, the plural kinds of the particulate materials are charged from the charging port at the top of the mixing apparatus of the present invention at the same time. Then, the mixed particulate materials are obtained from the discharging port at the bottom of the mixing apparatus of the present invention.

The step of humidifying the space for mixing the particulate materials in the mixing apparatus of the present invention by the humidifying means is conducted so that the space where the particulate materials are to be mixed is humidified. The term "humidify" means that a relative humidity in the space is made at 50% or more. When using the humidifier as the humidifying means, this step is conducted by supplying humidified air from the humidifier to the space for mixing the particulate materials during a period, which is generally 5 to 15 minutes, before the next step is conducted. However, it could be depend on conditions such as ability of the humidifier used, and a size of the space for mixing the particulate materials.

The step of charging the plural kinds of the particulate materials at the same time from the charging port at the top of the mixing apparatus of the present invention is conducted by, for example, charging the plural kinds of the particulate materials which are measured to give a desired mixing ratio, from the top of the mixing apparatus of the present invention at the same time. The measurement and charge of the particulate materials may be carried out by hands, but generally conducted by using an automated weighing instrument and a storage tank having a damper which is able to be opened or closed automatically. The storage tank may have a structure of a general type like a hopper, or may have its inside structure separated into plural chambers of which outlets are directed to different directions.

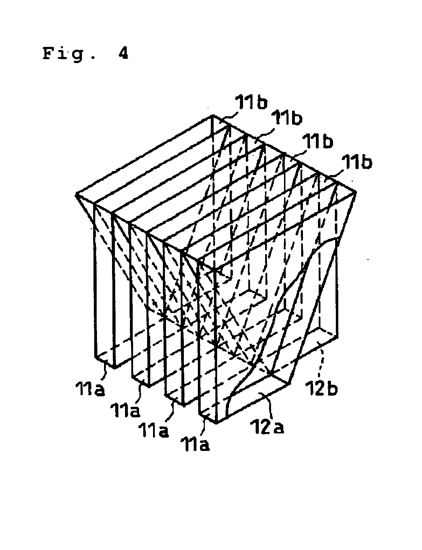

One specific structure of the storage tank is illustrated in Fig. 4. The storage tank shown in Fig. 4 has an inside structure in which thin-shaped plural funnel parts 11a and 11b are located alternately. The funnel parts 11a and 11b are arranged so that their apertures as outlets 12a and 12b are at opposite sides.

In general, a chute for collecting the particulate materials after being mixed and a damper if necessary are located, and the mixed particulate materials are collected by the chute and discharged into a packaging bag or the like located below the chute. Since each of the particulate materials is measured to give a desired ratio before being charged into the mixing apparatus of the present invention, the particulate materials packed in the bag are in a mixture of the plural kinds of the particulate materials.

The particulate materials to be mixed according to the present invention comprise as at least one of the plural kinds of particulate materials, for example, but not limited to, a particulate material containing a pharmaceutical component, an agrochemical component or a fertilizer component.

With respect to the mixing ratio of the mixture of the particulate materials produced by the mixing apparatus of the present invention, a ratio of one kind of the particulate materials is 1 to 99% by weight in the total weight of the obtained mixture of the particulate materials. When the mixture of the particulate materials produced by the mixing apparatus of the present invention is composed of two kinds of particulate materials, the ratio of them is generally 50:50 to 1:99, and preferably 50:50 to 75:25 by weight. A particle diameter of the particulate material to be mixed by the mixing apparatus of the present invention is generally 0.2-20 mm, and preferably 0.3-10 mm as a volume median diameter. Number of particles in one gram of the particulate material to be mixed by the mixing apparatus of the present invention is generally 50-5000 particles, and preferably 200-3000 particles. An apparent specific gravity of the particulate material to be mixed by the mixing apparatus of the present invention is generally 0.3-1.5 g/ml, and preferably 0.7-1.2 g/ml. The apparent specific gravity can be measured according to the Zen-Noh method. A difference in the apparent specific gravity between two or more kinds of the particulate materials to be mixed by the present invention is preferably 0.3 g/ml or less, and more preferably 0.2 g/ml or less. A shape of the particulate material is generally cubic, rectangular, trigonal pyramidal, circular conic, circular cylindrical, spherical, dumbbell-like, ellipsoidal, oval, convex conic, concave conic, plate-like or the like.

When the particulate material used in the present invention contains an agrochemical component, this particulate material is generally made of the agrochemical component and a carrier (or support) by shaping after adding thereto one or more additives such as a surfactant, a binder, a solvent, a stabilizing agent, a coloring agent, a covering agent and so on, if necessary.

The agrochemical component may comprise an insecticide, a fungicide, a herbicide, an insect growth regulator, a plant growth regulator and so on, and examples may comprise following compounds:
organophosphorus compounds such as O,O-dimethyl-O-(3-methyl-4-nitrophenyl) phosphorothioate, O,O-dimethyl-O-(3-methyl-4-(methylthio)phenyl) phosphorothioate, O,O-diethyl-0-2-isopropyl-6-methylpyrimidin-4-yl phosphorothioate, O,O-diethyl-0-3,5,6-trichloro-2-pyridyl phosphorothioate, O,S-dimethyl acetylphosphoramidothioate, S-2,3-dihydro-5-methoxy-2-oxo-1,3,4-thiadiazol-3-ylmethyl 0,0-dimethyl phosphorodithioate, O,O-diethyl S-2-ethylthioethyl phosphorodithioate, 2,2-dichlorovinyl dimethyl phosphate, O-ethyl O-4-(methylthio)phenyl S-propyl phosphorodithioate, O-4-cyanophenyl O,O-dimethyl phosphorothioate, 2-methoxy-4H-1,3,2-benzodioxaphosphorine-2-sulfide, O,O-dimethyl-S-(N-methylcarbamoylmethyl) dithiophosphate, ethyl 2-dimethoxyphosphinothioylthio(phenyl)acetate, diethyl (dimethoxyphosphinothioylthio) succinate, dimethyl 2,2,2-trichloro-1-hydroxyethylphosphonate, S-3,4-dihydro-4-oxo-1,2,3-benzotriazin-3-ylmethyl 0,0-dimethyl phosphorodithioate, dimethyl-{(E)-1-methyl-2-(methylcarbamoyl)vinyl} phosphate, O,O,O',O'-tetraethyl-S,S'-methylenebis(phosphorodithioate) and so on; carbamate compounds such as 2-sec-butylphenyl methylcarbamate, ethyl N-{2,3-dihydro-2,2-dimethylbenzofuran-7-yloxycarbonyl(methyl)aminothio}-N-isopropyl-β-alaninate, 2-isopropoxyphenyl-N-methylcarbamate, 2,3-dihydro-2,2-dimethyl-7-benzo[b]furanyl N-dibutylaminothio-N-methylcarbamate, 1-naphthyl-N-methylcarbamate, S-methyl-N-(methylcarbamoyloxy) thioacetimidate, 2-(ethylthiomethyl)phenyl methylcarbamate, 2-methyl-2-(methylthio)propionaldehyde O-methylcarbamoyloxime, N,N-dimethyl-2-methylcarbamoyloxyimino-2-(methylthio) acetamide, S-4-phenoxybutyl-N,N-dimethylthiocarbamate and so on; pyrethroid compounds such as 2-(4-ethoxyphenyl)-2-methyl-1-(3-phenoxybenzyl)oxypropane, (RS)-α-cyano-3-phenoxybenzyl (RS)-2-(4-chlorophenyl)-3-methylbutyrate, (S)-α-cyano-3-phenoxybenzyl (S)-2-(4-chlorophenyl)-3-methylbutyrate, (RS)-α-cyano-3-phenoxybenzyl 2,2,3,3-tetramethylcyclopropanecarboxylate, (RS)-α-cyano-3-phenoxybenzyl (1RS)-cis,trans-3-(2,2-dichlorovinyl)-2,2-dimethylcyclopropanecarboxylate, 3-phenoxybenzyl (1RS)-cis,trans-3-(2,2-dichlorovinyl)-2,2-dimethylcyclopropanecarboxylate, (RS)-α-cyano-3-phenoxybenzyl (1RS,3Z)-cis-3-(2-chloro-3,3,3-trifluoroprop-1-enyl)-2,2-dimethylcyclopropanecarboxylate, (S)-α-cyano-3-phenoxybenzyl (1R)-cis-3-(2,2-dibromovinyl)-2,2-dimethylcyclopropanecarboxylate, (RS)-α-cyano-3-phenoxybenzyl (RS)-2,2-dichloro-1-(4-ethoxyphenyl) cyclopropanecarboxylate, α-cyano-3-phenoxybenzyl N-(2-chloro-α,α,α-trifluoro-p-tolyl)-D-valinate, 2-methyl-3-phenylbenzyl (1RS,3Z)-cis-3-(2-chloro-3,3,3-trifluoro-1-propenyl)-2,2-dimethylcyclopropanecarboxylate, 2-(4-bromodifluoromethoxyphenyl)-2-methyl-1-(3-phenoxybenzyl) methylpropane, (S)-α-cyano-3-phenoxybenzyl (1R)-cis-3-(1,2,2,2-tetrabromoethyl)-2,2-dimethylcyclopropanecarboxylate, (4-ethoxyphenyl)-{3-(4-fluoro-3-phenoxyphenyl)propyl}dimethylsilane, 3-phenoxybenzyl (1R)-cis,trans-2,2-dimethyl-3-(2-methyl-1-propenyl)cyclopropanecarboxylate, (RS)-α-cyano-3-phenoxybenzyl (1R)-cis,trans-2,2-dimethyl-3-(2-methyl-1-propenyl)cyclopropanecarboxylate, 5-benzyl-3-furylmethyl (1R)-cis,trans-2,2-dimethyl-3-(2-methyl-1-propenyl)cyclopropanecarboxylate, (S)-α-cyano-3-phenoxybenzyl (1R,3Z)-cis-(2,2-dimethyl-3-{3-oxo-3-(1,1,1,3,3,3-hexafluoropropyloxy)propenyl}cyclopropanecarboxylate, (RS)-α-cyano-4-fluoro-3-phenoxybenzyl 3-(2,2-dichlorovinyl)-2, 2-dimethylcyclopropanecarboxylate, 2,3,5,6-tetrafluoro-4-methylbenzyl (1RS,3Z)-cis-3-(2-chloro-3,3,3-trifluoro-1-propenyl)-2,2-dimethylcyclopropanecarboxylate, 2,3,5,6-tetrafluorobenzyl (1R)-trans-3-(2,2-dichlorovinyl)-2,2-dimethylcyclopropanecarboxylate, 3,4,5,6-tetrahydrophthalimidomethyl (1RS)-cis,trans-2,2-dimethyl-3-(2-methyl-1-propenyl) cyclopropanecarboxylate, (RS)-2-methyl-4-oxo-3-(2-propenyl)-2-cyclopenten-1-yl (1RS)-cis,trans-2,2-dimethyl-3-(2-methyl-1-propenyl) cyclopropanecarboxylate, (S)-2-methyl-4-oxo-3-(2-propynyl)-2-cyclopenten-1-yl (1R)-cis,trans-2,2-dimethyl-3-(2-methyl-1-propenyl) cyclopropanecarboxylate, (RS)-1-ethynyl-2-methyl-2-pentenyl (1R)-cis,trans-2,2-dimethyl-3-(2-methyl-1-propenyl)cyclopropanecarboxylate, 2,5-dioxo-3-(2-propynyl)imidazolidin-1-ylmethyl (1R)-cis,trans-2,2-dimethyl-3-(2-methyl-1-propenyl)cyclopropanecarboxylate, 5-(2-propynyl)furfuryl (1R)-cis,trans-2,2-dimethyl-3-(2-methyl-1-propenyl)cyclopropanecarboxylate, 5-(2-propynyl)furfuryl 2,2,3,3-teramethylcyclopropanecarboxylate and so on; thiadiazine derivatives such as 2-tert-butylimino-3-isopropyl-5-phenyl-1,3,5-thiadiazin-4-one and so on; nitroimidazolidine derivatives; nereistoxin derivatives such as S,S'-(2-dimethylaminotrimethylene)bis(thiocarbamate), N,N-dimethyl-1,2,3-trithian-5-ylamine, S,S'-2-dimethylaminotrimethylenedi(benzenethiosulfonate) and so on; N-cyanoamidine derivatives such as N-cyano-N'-methyl-N'-(6-chloro-3-pyridylmethyl)acetamidine and so on; chlorinated hydrocarbon compounds such as 6,7,8,9,10,10-hexachloro-1,5,5a,6,9,9a-hexahydro-6,9-methano-2,4,3-benzodioxathiepine oxide, 1,2,3,4,5,6-hexachlorocyclohexane, 1,1-bis(4-chlorophenyl)-2,2,2-trichloroethanol and so on; benzoylurea compounds such as 1-{3,5-dichloro-4-(3-chloro-5-trifluromethylpyridin-2-yloxy)phenyl}-3-(2,6-difluorobenzoyl)urea, 1-(3,5-dichloro-2,4-difluorophenyl)-3-(2,6-difluorobenzoyl)urea, 1-{4-(2-chloro-4-trifluromethylphenoxy)-2-fluorophenyl}-3-(2,6-difluorobenzoyl)urea and so on; formamidine derivatives such as N,N'-{(methylimino)dimethylidine}-di-2,4-xylidine, N'-(4-chloro-2-methylphenyl)-N,N-dimethylmethinimidamide and so on; thiourea derivatives such as N-(2,6-diisopropyl-4-phenoxyphenyl)-N'-t-butylcarbodiimide and so on; N-phenylpyrazole compounds; 5-methoxy-3-(2-methoxyphenyl)-1,3,4-oxadiazol-2-(3H)-one; isopropyl 4,4'-dibromobenzilate; 4-chlorophenyl 2,4,5-trichlorophenyl sulfone; S,S-6-methylquinoxaline-2,3-diyl dithiocarbonate; 2-(4-tert-butylphenoxy) cyclohexylprop-2-yl sulfite; bis{tris(2-methyl-2-phenylpropyl)tin} oxide; (4RS,5RS)-5-(4-chlorophenyl)-N-chlorohexyl-4-methyl-2-oxo-1,3-thiazolidine-3-carboxamide; 3,6-bis(2-chlorophenyl)-1,2,4,5-tetrazine; 2-tert-butyl-5-(4-tert-butylbenzylthio)-4-chloropyridazin-3(2H)-one; tert-butyl (E)-4-[(1,3-dimethyl-5-phenoxypyrazol-4-yl) methyleneaminooxymethyl]benzoate; N-(4-tert-butylbenzyl)-4-chloro-3-ethyl-1-methyl-5-pyrazolecarboxamide; 5-chloro-N-[2-{4-(2-ethoxyethyl)-2,3-dimethylphenoxy}ethyl]-6-ethylpyrimidin-4-amine; 5-methyl[1,2,4]triazolo[3,4-b]benzothiazole; methyl 1-(butylcarbamoyl)benzimidazole-2-carbamate; 6-(3,5-dichloro-4-methylphenyl)-3(2H)-pyridazinone; 1-(9-chlorophenoxy)-3,3-dimethyl-1-(1H-1,2,4-triazol-1-yl)butanone; (E)-4-chloro-2-(trifluoromethyl)-N-[1-(imidazol-1-yl)-2-propoxyethylidene]aniline; 1-[N-propyl-N- [2- (2, 4, 6-trichlorophenoxy)ethyl]carbamoyl]imidazole; (E)-1-(4-chlorophenyl)-4,4-dimethyl-2-(1H-1,2,4-triazol-1-yl)-1-penten-3-ol; 1-(4-chlorophenyl)-4,4-dimethyl-2-(1H-1,2,4-triazol-1-yl)pentan-3-ol; (E)-1-(2,4-dichlorophenyl)-4,4-dimethyl-2-(1H-1,2,4-triazol-1-yl)-1-penten-3-ol; 1-(2,4-dichlorophenyl)-4,4-dimethyl-2-(1H-1,2,4-triazol-1-yl)pentan-3-ol; 4-[3-(4-tert-butylphenyl)-2-methylpropyl]-2,6-dimethylmorphorine; 2-(2,4-dichlorophenyl)-1-(1H-1,2,4-triazol-1-yl)hexan-2-ol; 0,0-diethyl O-2-quinoxalinyl phosphorothioate; O-(6-ethoxy-2-ethyl-4-pyrimidinyl) O,O-dimethyl phosphorothioate; 2-diethylamino-5,6-dimethylpyrimidin-4-yl dimethylcarbamate; 4-(2,4-dichlorobenzoyl)-1,3-dimethyl-5-pyrazolyl p-toluenesulfonate; 4-amino-6-(1,1-dimethyethyl)-3-methylthio-1,2,4-triazin-5(4H)-one; 2-chloro-N-[(4-methoxy-6-methyl-1,3,5-triazin-2-yl)aminocarbonyl]benzenesulfonamide; 2-methoxycarbonyl-N-[(4,6-dimethoxypyrimidin-2-yl)aminocarbonyl]benzenesulfonamide; 2-methoxycarbonyl-N-[(4,6-dimethylpyrimidin-2-yl)aminocarbonyl]benzenesulfonamide; 2-methoxycarbonyl-N-[(4-methoxy-6-methyl-1,3,5-triazin-2-yl)aminocarbonyl] benzenesulfonamide; 2-ethoxycarbonyl-N-[(4-chloro-6-methoxypyrimidin-2-yl)aminocarbonyl]benzenesulfonamide; 2-(2-chloroethoxy)-N-[(4-methoxy-6-methyl-1,3,5-triazin-2-yl)aminocarbonyl]benzenesulfonamide; 2-methoxycarbonyl-N-[(4,6-dimethoxypyrimidin-2-yl)aminocarbonyl]phenylmethanesulfonamide; 2-methoxycarbonyl-N-[(4-methoxy-6-methyl-1,3,5-triazin-2-yl)aminocarbonyl] thiophene-3-sulfonamide; 4-ethoxycarbonyl- N-[(4,6-dimethoxypyrimidin-2-yl)aminocarbonyl]-1-methylpyrazole-5-sulfonamide; 2-[4,5-dihydro-4-methyl-4-(1-methylethyl)-5-oxo-1H-imidazol-2-yl]-3-quinolinecarboxylic acid; 2-[4,5-dihydro-4-methyl-4-(1-methylethyl)-5-oxo-1H-imidazol-2-yl]-5-ethyl-3-pyridinecarboxylic acid; methyl 6-(4-isopropyl-4-methyl-5-oxoimidazolin-2-yl)-m-toluate; methyl 2-(4-isopropyl-4-methyl-5-oxoimidazolin-2-yl)-p-toluate; 2-(4-isopropyl-4-methyl-5-oxoimidazolin-2-yl)nicotinic acid; N-(4-chlorophenyl)methyl-N-cyclopentyl-N'-phenylurea; (RS)-2-cyano-N-((R)-1(2,4-dichlorophenyl) ethyl)-3,3-dimethylbutylamide; N-(1,3-dihydro-1,1,3-trimethylisobenzofurane-4-yl)-5-chloro-1,3-dimethylpyrazole-4-carboxyamide; N-(2,6-dibromo-4-(trifluoromethoxy)phenyl)-2-methyl-4-(trifluoromethyl)-5-thiazolecarboxyamide; 2,2-dichloro-N-(1-(4-chlorophenyl) ethyl) -3-methylcyclopropanecarboxyamide; methyl (E)-2-2-6-(2-cyanophenoxy)pyrimidine-4-yloxy-phenyl-3-methoxyacrylate; 5-methyl-1,2,4-triazoro(3,4-b)benzothiazole; 3-allyloxy-1,2-benzoisothiazole-1,1-dioxide; diisipropyl=1,3-dithiorane-2-ylidene-malonate; and O,O-diisopropyl-O-4-methylthiophenylphosfate.

When the particulate material used in the present invention contains the agrochemical component, this particulate material is generally made of the agrochemical component supported by a following carrier. The carrier can be a mineral carrier, a vegetable carrier, an animal carrier, a synthetic carrier and so on. Examples of the mineral carrier include kaolin minerals such as kaolinite, dickite, nacrite and halloysite; serpentinites such as chrysotile, lizardite, antigorite and amesite; montmorillonite minerals such as sodium montmorillonite, calcium montmorillonite and magnesium montmorillonite; smectites such as saponite, hectorite, sauconite and beidellite; micas such as pyrophyllite, talc, agalmatolite, muscovite, phengite, sericite and illite; silicas such as cristobalite and quartz; hydrated magnesium silicates such as attapulgite and sepiolite; calcium carbonates such as dolomite and calcium carbonate fine powder; sulfate minerals such as gypsum and plaster; zeolite; boiling stone; tuff; vermiculite; laponite; pumice; diatomite; acidic clay; activated clay and so on. Examples of the vegetable carrier include cellulose, hull, wheat flour, wood flour, starch, bran, wheat bran, soy bean flour and so on. Examples of the synthetic carrier include wet silica, dry silica, calcinated product of wet silica, surface modified silica, processed starch (for example, Pineflow manufactured by Matsutani Kagaku K.K., Japan) and so on. Such carrier generally makes up 0.5 to 99.9% by weight, and preferably 25 to 99.5% by weight of the particulate material.

When the particulate material used in the present invention contains a fertilizer component, this particulate material generally contains the fertilizer component and made by shaping after adding thereto one or more additives such as a binder, a stabilizing agent, a coloring agent, a covering agent and so on, if necessary.

Examples of the fertilizer component include urea, ammonium sulfate, ammonium chloride, ammonium phosphate, ammonium nitrate, lime nitrogen, sodium nitrate, acetaldehyde condensed urea, calcined phosphate, processed phosphate fertilizer, concentrated superphosphate, phosphate fertilizer mixture, potassium chloride, potassium sulfate magnesia, potassium bicarbonate, potassium silicate, potassium phosphate, potassium nitrite and so on.

When at least one kind of the particulate materials used in the present invention contains a pharmaceutical component, an agrochemical component or a fertilizer component, the particulate material containing the pharmaceutical component, the agrochemical component or the fertilizer component may be a coated particulate material which is coated with a resin. The resin in the coating can be, for example, a wax, a water-soluble polymer, a thermoplastic resin, a thermoset resin and so on.

Examples of the wax include synthetic waxes such as carbowax, Hoechst wax, sucrose ester and fatty acid ester; natural waxes such as carnauba wax, beeswax and Japan wax; petroleum waxes such as paraffin wax, petrolactam and so on.

Examples of the thermoplastic resin include polyolefins such as polyethylene, polypropylene, polybutene and polystyrene; vinyl polymers such as polyvinyl acetate, polyvinyl chloride, polyvinylidene chloride, polyacrylic acid, polymethacrylic acid, polyacrylic ester and polymethacrylic ester; diene polymers such as butadiene polymer, isoprene polymer, chloroprene polymer, butadienestyrene copolymer, ethylene-propylene-diene copolymer and styrene-isoprene copolymer; polyolefin copolymers such as ethylene-propylene copolymer, butene-ethylene copolymer, butene-propylene copolymer, ethylene-vinyl acetate copolymer, ethylene-acrylic acid copolymer, ethylene-methacrylic acid copolymer, ethylene-methacrylic ester copolymer, ethylene-carbon monoxide copolymer and ethylene-vinyl acetate-carbon monoxide copolymer; vinyl chloride copolymers such as vinyl chloride-vinyl acetate copolymer and vinylidene chloride-vinyl chloride copolymer; and so on.

Examples of the thermoset resin include urethane resin, epoxy resin, alkyd resin, unsaturated polyester resin, phenol resin, urea resin, melamine resin, silicone resin and so on.

Urethane resin is usually generated by a reaction of polyisocyanate and polyol in the presence of a curing agent such as an organic metal or amine. Polyisocyanate and polyol as monomers of the urethane resin are generally used alone as the monomers, or in the form of a solution, a water-based emulsion, an organic solvent-based emulsion or the like. Examples of the polyisocyanate include toluene diisocyanate (TDI), diphenylmethane diisocyanate (MDI), naphthalene diisocyanate, tolidine diisocyanate, hexamethylene diisocyanate, isophorone diisocyanate, xylylene diisocyanate, 4,4-methylenebis(cyclohexylisocyanate), trimethylhexamethylenediisocyanate, 1,3-(isocyanatemethyl)cyclohexane, triphenylmethanetriisocyanate, tris(isocyanataphenyl)thiophosphate, and a mixture thereof. In place of the polyisocyanate monomer, modified one or an oligomer of the above exemplified polyisocyanate can be used. Examples of the modified polyisocyanate include adducts of diisocyanate, biuret condensation products of three molecules of diisocyanate, isocyanurate condensation products of diisocyanate, isocyanate prepolymers, two molecules condensation products of diisocyanate and so on. Examples of the polyol include condensation type polyester polyol, polyether polyol, poly(meta)acrylic acid polyol, lactone type polyether polyol, polycarbonate polyol, natural polyol and modified one thereof. Condensation type polyester polyol is usually generated by a condensation reaction of polyol and dibasic acid, and polyether polyol is usually generated by a polymerization reaction of cyclic oxide. Poly(meta)acrylic acid polyol is usually generated by a condensation reaction of poly (meta) acrylic acid and polyol, a condensation reaction of (meta)acrylic acid and polyol, or a polymerization reaction of (meta)acrylic ester monomer. Lactone type polyether polyol can be obtained by a ring-opening polymerization of ε-caprolactam with an initiating reagent of polyvalent alcohol. Polycarabonate polyol is usually generated by a reaction of glycol and carbonate. Examples of the polyol include methylene glycol, ethylene glycol, propylene glycol, tetramethylene glycol, hexamethylene diol, trimethylol propane, poly(tetramethylene glycol), glycerin, pentaerythritol, sorbitol, sucrose and oligomers thereof. The dibasic acid may be adipic acid or phthalic acid. As the (meta)acrylic acid, acrylic acid, methacrylic acid or the like is generally used.

Epoxy resin is usually generated by a reaction of phenol or alcohol with epichlorohydrin in the presence of a curing agent, a reaction of carboxylic acid with epichlorohydrin in the presence of a curing agent, a reaction of amine, cyanuric acid or hydantoin with epichlorohydrin in the presence of a curing agent, a reaction of an aliphatic cyclic epoxy compound in the presence of a curing agent such as peracetic acid, and so on. Examples of the epoxy resin include glycidyl ether type epoxy resins such as bisphenol A type, bisphenol F type, brominated bisphenol A type, hydrogenated bisphenol A type, bisphenol S type, bisphenol AF type, biphenyl type, naphthalene type, fluorene type, phenol novolac type, orthocresol novolac type, DPP novolac type, trishydroxyphenylmethane type, tetraphenylolethane type; glycidyl amine type epoxy resins such as tetraglycidyldiaminodiphenylmethane type, triglycidylisocyanurate type, hydantoin type, aminophenol type, aniline type, toluidine type; alicyclic type epoxy resins, and so on.

Alkyd resin is, for example, generated by a reaction of polybasic acid with polyvalent alcohol in the presence of, if necessary, a modifying agent such as natural vegetable oil or animal fat, a metallic soap, or an antiskinning agent. Examples of the polybasic acid include phthalic anhydride, maleic anhydride and so on, and examples of the polyvalent alcohol include pentaerythritol, glycerin and so on. Examples of the modifying agent include, for example, soybean oil, linseed oil, tung oil, safflower oil, coconut oil, palm oil, dehydrated castor oil and so on. Examples of the metallic soap usually include metal salts of naphthenic acid or octylic acid such as manganese, cobalt, zirconium, nickel, iron, and lead salts, for example, zirconium octylate, manganese naphthenate, cobalt octylate, and mixtures thereof. Examples of the antiskinning agent include dipentene, methoxyphenol, cyclohexanone oxime, methyl ethyl ketone oxime, and mixtures thereof.

Unsaturated polyester resin is usually obtained by a reaction of unsaturated dibasic acid with divalent alcohol in the presence of vinyl monomer. Examples of the unsaturated dibasic acid include phthalic anhydride, isophthalic acid, terephthalic acid, succinic acid, adipic acid, azelaic acid, sebacic acid, tetrahydrophthalic anhydride, hexahydrophthalic anhydride, tetrabromophthalic anhydride, tetrachlorophthalic anhydride, HEX® acid anhydride (i.e. chlorendic anhydrate), endomethylenetetrahydrophthalic anhydride, and so on. Examples of the divalent alcohol include ethylene glycol, propylene glycol, 1,3-butylene glycol, 1,6-hexanediol, diethylene glycol, dipropylene glycol, neopentyl glycol, triethylene glycol, hydrogenated bisphenol A, bisphenol dihydroxypropyl ether, and so on. Examples of the vinyl monomer include styrene, vinyltoluene, chlorostyrene, diallyl phthalate, triallyl cyanurate, methyl methacrylate, and so on.

Phenol resin is generated by a reaction of a phenolic compound with aldehyde in the presence of a catalyst such as hydrochloric acid, oxalic acid, hexamethylenetetramine. Examples of the phenolic compound include phenol, o-cresol, m-cresol, p-cresol, xylenol, p-tert-butylphenol, resorcinol, and so on. From this reaction, novolac type phenol resins are obtained under an acidic catalyst condition, and resol type phenol resins are obtained under a basic catalyst condition.

Urea resin or melamine resin is usually generated by a reaction of urea or melamine with formaldehyde such as formalin in the presence of a basic catalyst.

In the present application, the particulate material containing the pharmaceutical component, the agrochemical component or the fertilizer component can be obtained by granulation according to a granulation method generally used for pharmaceutical preparation. As the granulation method, an extrusion granulation method, a compression granulation method, a mixing granulation method, a fluidized bed granulation method, a rolling motion granulation method, a coating granulation method and so on are used.

### Examples

Hereafter, effects of the mixing apparatus of the present invention is described with reference to examples in which plural kinds of the particulate materials are mixed by using the mixing apparatus of the present invention.

### [Particulate materials used for examples]

### Particulate Material A

Six parts by weight of an agrochemical component (1), 2 parts by weight of an agrochemical component (2), 2 parts by weight of a nonionic surfactant, 5 parts by weight of a binder, 1 part by weight of a stabilizing agent, 84 parts by weight of a mineral carrier were mixed and kneaded with an amount of water. Thus kneaded material was granulated by an extrusion granulator and dried to give a cylindrical particulate material A having a particle diameter of 500 to 1190 µm.

### Particulate Material B

Eight parts by weight of an agrochemical component (3), 2 parts by weight of a nonionic surfactant, 3 parts by weight of a binder, 13.5 part by weight of a water-soluble carrier, 73.5 parts by weight of a mineral carrier were mixed and kneaded with an amount of water. Thus kneaded material was granulated by an extrusion granulator and dried, and the obtained particulate material was coated with a urethane resin which was prepared from aromatic polyisocyanate and polyether polyol to give a cylindrical particulate material B having a particle diameter of 500 to 1190 µm.

### [Mixing apparatus used for examples]

A mixing apparatus shown in Fig. 5 was used. The mixing apparatus was made of SUS304. A storage tank shown in Fig. 4 was set on the top of this mixing apparatus, these two kinds of particulate materials ware charged thereto from the storage tank. The storage tank was provided on its funnel outlet with a damper which is able to be automatically opened and closed at an interval of 7 seconds. The particulate materials mixed were collected through a discharging chute and into a bag located below the damper.

Two ultrasonic humidifiers (each having a humidifying ability of 0.35 Liter/hr, available from Toyotomi Co., Ltd, Japan) were used as the humidifying means to supply humidified air into the mixing apparatus through washing windows above the combined position of the diffluent part and the confluent part in their first and third set of the mixing apparatus.

### [Example 1]

At first, the particulate materials were mixed under the condition in which the humidifier was not operated. In this case, a temperature inside the mixing apparatus was 7°C and a humidity inside the mixing apparatus was 44%.

Each of 500 g of the particulate material A and 500 g of the particulate material B were measured separately and charged to the storage tank in this order. Time of discharging the mixed particulate materials was determined in the vicinity of the damper below the discharging chute of the mixing apparatus. The time period from the beginning to the end of the discharge of the mixed particulate materials from the discharging chute of the mixing apparatus was 9 to 10 seconds per cycle.

Then, the humidifier was started to operate 10 minutes before the mixing of the particulate materials was started. The temperature inside the mixing apparatus was 4°C and the humidity inside the mixing apparatus was 91% just before the start of the mixing.

The mixing was carried out and the time of discharging the mixed particulate materials was determined similarly to the above. The time period from the beginning to the end of the discharge of the mixed particulate materials from the discharging chute of the mixing apparatus was about 7 seconds per cycle.

In the following examples, as similarly to Example 1, the mixing of the particulate materials was carried out and observed under the condition in which the humidifier was not operated (comparative examples), and the condition in which the humidifier was operated while the humidifier was started to operate 10 minutes before the mixing of the particulate materials was started (examples of the present invention).

### [Example 2]

### Condition in which the humidifier was not operated

The temperature inside the mixing apparatus 10°C, and the humidity inside the mixing apparatus 45%. The time period from the beginning to the end of the discharge of the mixed particulate materials was 9 to 10 seconds per cycle.

### • Condition in which the humidifier was operated

The temperature inside the mixing apparatus 10°C, and the humidity inside the mixing apparatus 53%. The time period from the beginning to the end of the discharge of the mixed particulate materials was about 7 seconds per cycle.

### [Example 3]

### • Condition in which the humidifier was not operated

The temperature inside the mixing apparatus 12°C, and the humidity inside the mixing apparatus 47%. The time period from the beginning to the end of the discharge of the mixed particulate materials was 9 to 10 seconds per cycle.

### • Condition in which the humidifier was operated

The temperature inside the mixing apparatus 10°C, and the humidity inside the mixing apparatus 61%. The time period from the beginning to the end of the discharge of the mixed particulate materials was about 7 seconds per cycle.

### [Example 4]

### • Condition in which the humidifier was not operated

The temperature inside the mixing apparatus 11°C, and the humidity inside the mixing apparatus 34%. The time period from the beginning to the end of the discharge of the mixed particulate materials was 9 to 10 seconds per cycle.

### Condition in which the humidifier was operated

The temperature inside the mixing apparatus 10°C, and the humidity inside the mixing apparatus 58%. The time period from the beginning to the end of the discharge of the mixed particulate materials was about 7 seconds per cycle.

### [Example 5]

### Condition in which the humidifier was not operated

The temperature inside the mixing apparatus 11°C, and the humidity inside the mixing apparatus 45%. The time period from the beginning to the end of the discharge of the mixed particulate materials was 9 to 10 seconds per cycle.

### Condition in which the humidifier was operated

The temperature inside the mixing apparatus 9°C, and the humidity inside the mixing apparatus 57%. The time period from the beginning to the end of the discharge of the mixed particulate materials was about 7 seconds per cycle.

### [Example 6]

### • Condition in which the humidifier was not operated

The temperature inside the mixing apparatus 8°C, and the humidity inside the mixing apparatus 36%. The time period from the beginning to the end of the discharge of the mixed particulate materials was 9 to 10 seconds per cycle.

### • Condition in which the humidifier was operated

The temperature inside the mixing apparatus 6°C, and the humidity inside the mixing apparatus 61%. The time period from the beginning to the end of the discharge of the mixed particulate materials was about 7 seconds per cycle.

### [Example 7]

### • Condition in which the humidifier was not operated

The temperature inside the mixing apparatus 9°C, and the humidity inside the mixing apparatus 38%. The time period from the beginning to the end of the discharge of the mixed particulate materials was 9 to 10 seconds per cycle.

### • Condition in which the humidifier was operated

The temperature inside the mixing apparatus 8°C, and the humidity inside the mixing apparatus 55%. The time period from the beginning to the end of the discharge of the mixed particulate materials was about 7 seconds per cycle.

### [Example 8]

### Condition in which the humidifier was not operated

The temperature inside the mixing apparatus 6°C, and the humidity inside the mixing apparatus 49%. The time period from the beginning to the end of the discharge of the mixed particulate materials was 9 to 10 seconds per cycle.

### • Condition in which the humidifier was operated

The temperature inside the mixing apparatus 5°C, and the humidity inside the mixing apparatus 66%. The time period from the beginning to the end of the discharge of the mixed particulate materials was about 7 seconds per cycle.

### Industrial Applicability

The mixing apparatus of the present invention can be used for mixing plural kinds of particulate materials with an improved efficiency.

## Claims

1. A mixing apparatus for mixing plural kinds of particulate materials by using a falling force, wherein the mixing apparatus comprises means (10) for humidifying a space for mixing the particulate materials, **characterized** that the humidifying means is adapted so that the relative humidity in the space for mixing is made at 50% or more.

2. The mixing apparatus according to Claim 1, **characterized in that** the mixing apparatus comprises at least one set of a diffluent part (1) for dividing a passage through which the particulate materials fall and a confluent part (2) for merging passages through which the particulate materials fall.

3. The mixing apparatus according to anyone of the preceding claims further comprising a charging port which is arranged at the top of the apparatus and a discharging port which is arranged at the bottom of the apparatus, wherein the space for mixing the particulate materials is arranged between the charging port and the discharging port.

4. The mixing apparatus according to anyone of the preceding claims wherein the humidifier means (10) is of a heating type, a steaming type, an ultrasonic type or a mixed type thereof.

5. A method for mixing plural kinds of particulate materials by using a falling force, wherein the method is carried out in a humidified space for mixing the particulate materials, **characterized in that** the relative humidity in the humidifying space is made at 50% or more.

6. The method according to Claim 5, **characterized in that** the plural kinds of the particulate materials comprise at least one kind of a particulate material containing a pharmaceutical component, an agrochemical component or a fertilizer component.

7. Method according to claim 5 or claim 6, **characterized in that** the humidified space is humidified by a humidifying means.

8. Method according to anyone of claims 5 to 7, **characterized in that** the method is carried out on an apparatus having a charging port which is arranged at the top of the apparatus and a discharging port which is arranged at the bottom of the apparatus as well as a space for mixing the particulate materials between the charging port and the discharging port.

9. Method according to anyone of claims 5 to 8, **characterized in that** a humidifier of the heating type, the steaming type, the ultrasonic type or a mixed type thereof is used for humidifying the humidifying space.

## Patentansprüche

1. Eine Mischvorrichtung zum Mischen mehrerer Arten teilchenförmiger Materialien unter Verwendung einer Fallkraft, wobei die Mischvorrichtung eine Einrichtung (10) zum Befeuchten eines Hohlraums zum Mischen der teilchenförmigen Materialien umfasst, **dadurch gekennzeichnet, dass** die Befeuchtungseinrichtung daran angepasst ist, dass die relative Feuchte in dem Hohlraum zum Mischen bei 50% oder mehr liegt.

2. Die Mischvorrichtung nach Anspruch 1, **dadurch gekennzeichnet, dass** die Mischvorrichtung mindestens einen Satz aus einem diffluenten Abschnitt (1) zur Unterteilung eines Durchlasses, durch den die teilchenförmigen Materialien fallen, und einen konfluenten Abschnitt (2) zum Zusammenführen von Durchlässen, durch die die teilchenförmigen Materialien fallen, umfasst.

3. Die Mischvorrichtung nach einem der vorhergehenden Ansprüche, weiter umfassend eine Beschickungsöffnung, die an der Oberseite der Vorrichtung angeordnet ist, und eine Auslassöffnung, die an der Unterseite der Vorrichtung angeordnet ist, wobei der Hohlraum zum Mischen der teilchenförmigen Materialien zwischen der Beschickungsöffnung und der Auslassöffnung angeordnet ist.

4. Die Mischvorrichtung nach einem der vorhergehenden Ansprüche, wobei die Befeuchtungseinrichtung (10) eine Einrichtung vom Erwärmungstyp, Dampftyp, Ultraschalltyp oder ein Mischtyp davon ist.

5. Ein Verfahren zum Mischen mehrerer Arten teilchenförmiger Materialien unter Verwendung einer Fallkraft, wobei das Verfahren in einem befeuchteten Hohlraum zum Mischen der teilchenförmigen Materialien durchgeführt wird, **dadurch gekennzeichnet, dass** die relative Feuchte in dem Befeuchtungshohlraum bei 50% oder mehr liegt.

6. Das Verfahren nach Anspruch 5, **dadurch gekennzeichnet, dass** die mehreren Arten der teilchenförmigen Materialien mindestens eine Art eines teilchenförmigen Materials umfassen, das einen pharmazeutischen Bestandteil, einen agrochemischen Bestandteil oder einen Düngemittel-Bestandteil enthält.

7. Verfahren nach Anspruch 5 oder Anspruch 6, **dadurch gekennzeichnet, dass** der befeuchtete Hohlraum durch eine Befeuchtungseinrichtung befeuchtet wird.

8. Verfahren nach einem der Ansprüche 5 bis 7, **dadurch gekennzeichnet, dass** das Verfahren auf einer Vorrichtung mit einer Beschickungsöffnung, die an der Oberseite der Vorrichtung angeordnet ist, und einer Auslassöffnung, die an der Unterseite der Vorrichtung angeordnet ist, sowie einem Hohlraum zum Mischen der teilchenförmigen Materialien zwischen der Beschickungsöffnung und der Auslassöffnung durchgeführt wird.

9. Verfahren nach einem der Ansprüche 5 bis 8, **dadurch gekennzeichnet, dass** ein Befeuchter vom Erwärmungstyp, Dampftyp, Ultraschalltyp oder einem Mischtyp davon zur Befeuchtung des Befeuchtungshohlraumes verwendet wird.

## Revendications

1. Appareil de mélange pour mélanger plusieurs types de matières particulaires à l'aide d'une force de chute, dans lequel l'appareil de mélange comprend un moyen (10) pour humidifier un espace destiné à mélanger les matières particulaires, **caractérisé en ce que** le moyen d'humidification est conçu pour que l'humidité relative dans l'espace de mélange soit amenée à 50 % ou plus.

2. Appareil de mélange selon la revendication 1, **caractérisé en ce que** l'appareil de mélange comprend au moins un ensemble d'une partie à flux divergent (1) pour diviser un passage à travers lequel les matières particulaires chutent et une partie à flux convergent (2) pour fusionner les passages à travers lesquels les matières particulaires chutent.

3. Appareil de mélange selon l'une quelconque des revendications précédentes, comprenant en outre un orifice de chargement qui est agencé au sommet de l'appareil et un orifice d'évacuation qui est agencé à la base de l'appareil, dans lequel l'espace de mélange des matières particulaires est agencé entre l'orifice de chargement et l'orifice d'évacuation.

4. Appareil de mélange selon l'une quelconque des revendications précédentes, dans lequel le moyen d'humidificateur (10) est de type chauffant, de type à vapeur, de type à ultrasons ou d'un type mixte de ceux-ci.

5. Procédé de mélange de plusieurs types de matières particulaires à l'aide d'une force de chute, dans lequel le procédé est réalisé dans un espace humidifié pour mélanger les matières particulaires, **caractérisé en ce que** l'humidité relative dans l'espace humidification est amenée à 50 % ou plus.

6. Procédé selon la revendication 5, **caractérisé en ce que** les plusieurs types de matières particulaires comprennent au moins un type de matière particulaire contenant un composant pharmaceutique, un composant agrochimique ou un composant d'engrais.

7. Procédé selon la revendication 5 ou la revendication 6, **caractérisé en ce que** l'espace humidifié est humidifié par un moyen d'humidification.

8. Procédé selon l'une quelconque des revendications 5 à 7, **caractérisé en ce que** le procédé est réalisé sur un appareil comportant un orifice de chargement qui est agencé au sommet de l'appareil et un orifice d'évacuation qui est agencé à la base de l'appareil, ainsi qu'un espace pour mélanger les matières particulaires entre l'orifice de chargement et l'orifice d'évacuation.

9. Procédé selon l'une quelconque des revendications 5 à 8, **caractérisé en ce qu'**un humidificateur de type chauffant, de type à vapeur, de type à ultrasons ou d'un type mixte de ceux-ci est utilisé pour humidifier l'espace d'humidification.
